# EUROPEAN PATENT APPLICATION

(11) **EP 4 442 694 A1**
(43) Date of publication of application: **09.10.2024**
(21) Application number: 23806710.2
(22) Date of filing: 26.04.2023
(51) Int. Cl.: C07H 13/04, C07H 1/06

(54) **NEW CRYSTAL FORM OF TRISACCHARIDE**

(30) Priority: 17.05.2022 CN 202210533080
(71) Applicant: Shandong Henglu Biotech. Co., Ltd., Jinan, Shandong 250004 (CN)
(72) Inventor: YIN, Wencheng, Jinan, Shandong 250004 (CN); JIANG, Fujiao, Jinan, Shandong 250004 (CN); ZHU, Changxiong, Jinan, Shandong 250004 (CN); FANG, Xu, Jinan, Shandong 250004 (CN); HIROSE, Yoshihiko, Gifu 503-0097 (JP)
(74) Representative: Herrmann, Uwe
(86) International application number: PCT/CN2023/090965
(87) International publication number: WO 2023/221758

(57) **Abstract**

The present invention relates to a crystal of lacto-N-triaose, and belongs to the technical field of separation and purification. The technical solution of the present invention is a crystalline form B of LNTII, which crystalline form has characteristic diffraction peaks at positions of 2θ values (2θ±0.2°) of diffraction angles of 3.98, 6.98, 8.30, 9.60, 18.68, 19.25, 19.89 and 20.72 in the powder X-ray diffraction pattern. Provided in the present invention is a new crystalline form of lacto-N-triaose, thereby achieving the iteration of a technique for preparing LNTII and providing a reliable technique for industrial large-scale production.

## Description

The present invention claims priority to a Chinese patent application submitted to the China National Intellectual Property Administration on May 17, 2022, with its application number 202210533080.1 and the invention name "New Crystal Form of Trisaccharide". The complete disclosure of this application is incorporated by reference herein and forms a part of the present invention.

### TECHNICAL FIELD

The present invention relates to a crystal of lacto-N-triose, and belongs to the technical field of separation and purification.

### BACKGROUND

Human milk oligosaccharides (HMOs) are a collection of complex oligosaccharides composed of monosaccharides and derivatives, sialic acid and other structural units connected by glycosidic bonds, with high content and unique functions. Over 200 HMOs have been isolated from the human breast milk to date. However, understanding of HMOs has gone through more than a hundred years. As early as 1886, Australian pediatrician and microbiologist Escherich found a correlation between infant gut bacteria and their digestive function. In 1900, Moro and Tissier respectively confirmed differences of bacterial composition in the feces of breastfed and artificially fed infants. Was there any component in breast milk that causes different gut bacteria in infants? In 1926, Schönfeld discovered that the whey of breast milk contained factors that promoted the growth of bifidobacteria. In that way, what components in whey actually promoted the growth of bifidobacteria? In 1930, French scientists Polonowski and Lespagnol detected carbohydrate-like substances in breast milk whey, terming them "gynolactose." In 1954, György confirmed that the factor that promoted the growth of bifidobacteria, i.e., "bifidus factor", was actually oligosaccharides. In the same year, Polonowski and Montreuil separated oligosaccharides from gynolactose by using two-dimensional paper chromatography. In 1983, Egge et al. confirmed HMOs using fast-atom bombardment mass spectrometry. In 1999, Coppa et al. detected 30-50% of HMOs in the feces of breastfed infants in their original structural form. In 2000, it was reported that HMOs can be resistant to degradation by the digestive enzymes in the infant gastrointestinal tract, which fully demonstrates that the biological function of HMOs is not only to provide material and energy to infants in the ordinary sense.

Since the 21st century, with the advancement of research methods, it has been documented that the concentration of total HMOs (acidic and neutral oligosaccharides) in breast milk may decrease with a prolonged lactation period. Moreover, HMOs are of great significance for the growth and development of infants, as well as their current and long-term health outcomes. Specifically, HMOs can promote the balance of infant gut microbiota and the proliferation of beneficial bacteria in the intestine, inhibit the growth of harmful bacteria, resist pathogen infections, hinder the colonization of pathogenic bacteria, prevent inflammatory bowel disease and gastroenteritis, regulate the immune system, and promote cognitive development of infants. Therefore, timely supplementation of HMOs may be beneficial for maintaining the healthy growth of infants and young children. However, it is still a huge challenge for carbohydrate synthesis by synthetic workers. As for the reason, from the perspective of sugar chain synthesis in organisms, the synthesis of such molecules is not the replication of a single template, but is jointly regulated by a wide range of glycosyltransferases and glycosidases. It eventually determines the complexity, diversity, and micro-heterogeneity of the sugar chain structure. Fortunately, with the rapid development of glycochemistry and recombinant enzymology, major effects have been made by glycochemists and glycobiologists in the field of sugar synthesis, with successive reports of a series of new methods and strategies for synthesizing oligosaccharides.

Lacto-N-triose II (hereinafter referred to as LNTII, also known as GlcNAc-β1,3-Gal-β1,4-Glc, CAS: 75645-27-1, with the chemical structural formula shown in Formula 1).

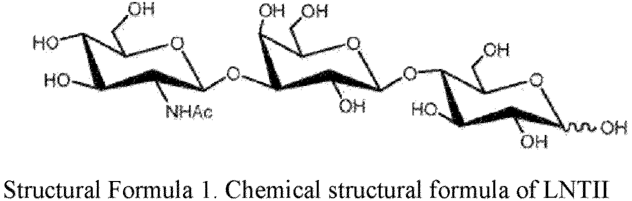

LNTII, main chain precursor of HMOs, is currently generally synthesized through chemical or biological methods. Exiting reports reveal that its solid form can be obtained by filtering and drying after evaporating and concentrating, freezing technology or spray-drying. In Chinese patent CN112154150A, HMOs are synthesized through enzymatic fermentation, followed by enzyme treatment, ultra-filtration, nano-filtration, passing through chromatographic column, decolorization, filtration for purification, and/or drying to obtain solid oligosaccharides. The proposed patent just provides a general method for obtaining the solid form, which, however, was verified by the applicant that the solid obtained was amorphous. Chinese patent CN109705175A records the method of purifying neutral HMOs from crude solution containing neutral HMOs, including the use of simulated moving bed chromatography and purification steps such as concentration, dialysis and/or filtration, and finally spray-drying to obtain amorphous powder particles with particle size of 5-500 microns. CN104428307A discloses a method for removing or at least significantly reducing the amount of organic solvent residue in HMOs, which includes the step of spray-drying of the aqueous solution of HMOs. However, it provides HMOs in the form of amorphous solids, accompanied by problems of high energy consumption and large material loss during spray-drying. In CN106132977A, the purified solution of fermentation broth is concentrated to a concentration of >1.5M and cooled to a temperature of <25°C, and <8°C more preferably, to obtain a crystal substance of neutral HMOs (Patent Specification [0055]). However, the specific crystalline form is not given in the Examples, with the description of the powdered amorphous particles obtained by spray-drying only. In CN109891173A, the organic solvent is removed from crystal oligosaccharides by exposing crystal oligosaccharide hydrates to water vapor. It is generally described that the hydrates contain crystal water, without mentioning the specific crystalline form of LNTII. Moreover, the method proposed in the above patent is time- and energy-consuming, which is not conducive to large-scale production. CN110483652A records the freeze-drying method for obtaining HMOs, but the freeze-dried powder (amorphous) obtained by freeze-drying technology also requires freezing and/or low temperature during its storage, transportation, and other processes, which increases the cost and difficulty of product preservation and use.

It is urgent to develop an efficient and large-scale industrial purification method for LNTII to provide stable and cost-effective solid forms of LNTII to the market and meet the needs of product iteration, and provide solid forms of LNTII at distinct advantages to accelerate its application in various products such as dairy products, food, pharmaceuticals, cosmetics and feed to meet the public needs.

### SUMMARY

The present invention aims to provide a new stable crystal of LNTII and its preparation method, thereby achieving the iteration of a separation technique for preparing LNTII and solid form, and providing a reliable technique for industrial large-scale production.

Through experiments, the applicant obtained a new crystal of LNTII and named it crystalline form B of LNTII, which is also referred to as crystalline form B in the present invention.

The technical solution of the present invention:
A crystalline form B of LNTII, with the characteristic diffraction peaks at positions of 2θ values (2θ±0.2°) of diffraction angles of 3.98, 6.98, 8.30, 9.60, 18.68, 19.25, 19.89 and 20.72 in the powder X-ray diffraction pattern.

Preferably, the crystalline form B described in the present invention, with the characteristic diffraction peaks at positions of 2θ values (2θ±0.2°) of diffraction angles of 3.98, 6.98, 8.30, 9.60, 11.62, 18.68, 19.25, 19.89, 20.72, 21.00 and 21.56 in the powder X-ray diffraction pattern.

Preferably, the crystalline form B in the present invention, with the characteristic diffraction peaks at positions of 2θ values (2θ±0.2°) of diffraction angles of 3.98, 6.98, 8.30, 9.60, 11.62, 12.72, 18.68, 19.25, 19.89, 20.72, 21.00, 21.56 and 29.06 in the powder X-ray diffraction pattern.

Preferably, the crystalline form B in the present invention, with the characteristic diffraction peaks at positions of 2θ values (2θ±0.2°) of diffraction angles of 3.98, 6.98, 8.30, 9.60, 11.62, 12.72, 18.68, 19.25, 19.89, 20.72, 21.00, 21.56, 26.98, 29.06, 29.54, 31.08, 31.84 and 33.36 in the powder X-ray diffraction pattern.

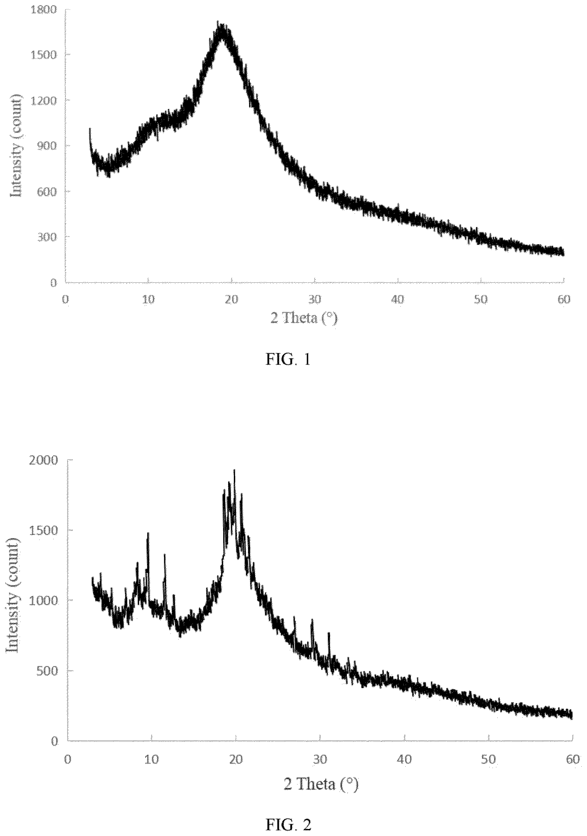

Preferably, the crystalline form B in the present invention, with its powder X-ray diffraction pattern shown in FIG. 2.

The melting point of the LNTII crystalline form B in the present invention ranges between 185-192°C; and the melting point of the LNTII amorphous powder disclosed by existing technology ranges between 130-134°C.

The procedure of preparing crystalline form B of LNTII comprises the following steps:
Step 1. Prepare a solution containing LNTII for further use.

Preferably, LNTII-containing solution is prepared at the concentration of between 0.2-0.5 g/mL;
Step 2. Cool down the temperature of LNTII-containing solution obtained in Step 1, preferably to a temperature of 25°C or below, and more preferably at a cooling rate of 0.5°C/min, avoiding cooling too fast; and a cooling rate of greater than 0.5°C/min will affect crystallization or the purity of the product.

Step 3. Stir and add an organic solvent to the solution in Step 2 for crystallization.

The preferred operation is to obtain LNTII-containing solution, for example, by dissolving LNTII in solid form, or by purifying or further concentrating the fermentation broth after fermentation and culture of LNTII-producing strains. The LNTII-producing strain herein is a strain known in this field that can produce LNTII through fermentation and culture, with known culture method in the field as well, as long as it can ferment the LNTII-producing strain to produce LNTII.

Preferably, ethanol is selected as the organic solvent, wherein the ethanol is anhydrous ethanol or ethanolic aqueous solution.

Preferably, the mass of the organic solvent in the solution shall be greater than that of water after the addition of organic solvent in this step. Preferably, the mass ratio of water and ethanol is adjusted at 1:2-5 (calculated as anhydrous ethanol); more preferably, at 1:3-4, and further preferably, at 1:3.5.

Preferably, stir and add ethanol or ethanolic aqueous solution to the solution in Step 2, with a fed-batch rate of 0.07-0.25 mL/min; and perform fed-batch cultivation of the crystal via stirring after the completion of fed-batch. Accelerated fed-batch process may affect crystallization or the purity of products.

Preferably, add crystal seeds in this step; and stir at a rate of over 100r/min during crystallization.

Preferably, continue stirring after the fed-batch of ethanol or ethanolic aqueous solution, and the crystallization time shall not be less than 12 h.

The preparation method of crystalline form B of LNTII herein includes separating the crystal in LNTII-containing solution, and the solvent of the solution contains water and ethanol.

Preferably, crystalline form B of LNTII of the present invention can be obtained by cooling and crystallizing after the addition of ethanol or ethanolic aqueous solution when LNTII-containing fermentation broth is purified through decolorization, desalination and other methods; and it can also be obtained by cooling and crystallizing after the addition of ethanol or ethanolic aqueous solution to the prepared aqueous solution by LNTII solid. The cooling and crystallizing described herein refer to cooling the prepared solution to a temperature below 25°C.

The lacto-N-triose II crystalline form B of the present invention can be used as an intermediate for synthesizing other sugars or compounds, and as a terminal product (e.g., a nutritional supplement for dairy products or food, etc.).

### Beneficial Effects:

The present invention provides a stable crystalline form B of LNTII and its preparation method, achieving the iteration of a technique for preparing LNTII and providing a reliable technique for industrial large-scale production. The crystalline form B and its preparation method herein have the following advantages compared to existing amorphous powders and preparation methods:
(1) The stability of crystalline form B of LNTII described in the present invention is superior to that of amorphous powder, which is beneficial for the long-term storage of LNTII products and their application in food and medicine. It can also reduce the packaging cost of LNTII and extend the shelf life of the product;
(2) The preparation method of crystalline form B herein is beneficial for the industrial application of separation and purification of LNTII products, reducing production costs and significantly improving production efficiency.

In summary, the present invention obtains LNTII crystalline form for the first time, achieving a historic breakthrough in the separation technology of HMOs and benefiting the general public.

### BRIEF DESCRIPTION OF THE DRAWINGS

FIG. 1. The X-ray diffraction pattern of lacto-N-triose II amorphous powder.
FIG. 2. The X-ray diffraction pattern of lacto-N-triose II crystalline form B.
FIG. 3. Scanning electron microscopy (SEM) images of lacto-N-triose II amorphous powder.
FIG. 4. SEM images of lacto-N-triose II crystalline form B.
FIG. 5. Morphology of lacto-N-triose II amorphous powder and crystalline form B before placing in a chamber with constant temperature and humidity (left: amorphous powder; and right: crystalline form B of LNTII).
FIG. 6. Morphology of lacto-N-triose II amorphous powder and crystalline form B after moisture absorption in a Constant Temperature & Humidity Chamber (at 25°C and relative humidity of 75%) (left: amorphous powder; and right: crystalline form B of LNTII).

### DETAILED DESCRIPTION

It shall be pointed out that the following explanations and embodiments are exemplary and are intended to provide further explanation of the present application. Unless otherwise specified, all technical and scientific terms used herein have the same meanings as those commonly understood by ordinary technical personnel in the technical field to which this application belongs.

The following part described herein provides further interpretative statement of the present invention through embodiments, but does not constitute a limitation of the present invention. It shall be understood that these embodiments are only used to explain the present invention and not to limit the scope of the present invention. The test methods without specific conditions stated in the following embodiments are commonly carried out under normal conditions.

In this invention, high-performance liquid chromatography (HPLC) is used to detect the content of LNTII under the following detection conditions:
Chromatographic column: Asahipak NH2P-504E 4.6 mm × 250 mm, 5 µm, mobile phase: acetonitrile: water=65: 35, flow rate: 1mL/min, detector: UV-VIS detector, wavelength: 210nm, column temperature: 30°C, sample volume: 10µL, and the running time of 30min.

In certain embodiments, the LNTII solid form is LNTII in the solid form commonly understood in the art, which can be prepared according to the public literature, can be the powder obtained by spray-drying, can be the solid obtained by freeze-drying powder or other methods. The invention is not limited to the way to obtain the LNTII solid form. LNTII fermentation broth is a fermentation broth obtained based on existing technology. For example, it can be prepared according to methods recorded in disclosed patents, such as the method described in Chinese patent CN108026556A with the invention name of "Production of human milk oligosaccharides in host microorganisms with modified import/export"; or according to the method recorded in Chinese patent CN115873051A. The above-mentioned patents in their entirety are incorporated herein by reference into the present invention herein and form a part of the present invention. However, it shall be noted that the present invention is not limited to the types of LNTII-producing strains, as long as they can produce LNTII through fermentation. Similarly, the method of strain fermentation and culture is easy to obtain and implement in this field. Additionally, the present invention is not limited to the fermentation and culture methods of LNTII-producing strains, as long as it can produce LNTII through fermentation and culture of LNTII-producing strains.

Specifically, for example, LNTII-producing strains can be prepared according to Example 1 of CN108026556A, including:
*Escherichia coli* BL21 (DE3) is selected as the host cell. Metabolic engineering includes deletion of specific genes and genomic integration of heterologous genes. Genomic deletion is performed according to the method described by Datsenko and Warner (Proc.Natl.Acad.Sci.USA 97:6640-6645(2000)). In order to prevent intracellular degradation of N-acetylglucosamine, genes encoding N-acetylglucosamine 6-phosphate deacetylase (nagA) and glucosamine 6-phosphate deaminase (nagB) are deleted in the genome of Escherichia coli BL21 (DE3). Genomic integration of heterologous genes is realized through EZ-Tn5 transposition. The N-acetylglucosamine transferase gene lgtA (GenBank: NP_274923) from *Neisseria meningitidis* MC58, which has been optimized for codons, is integrated into *Escherichia coli.*

LNTII-producing strains are cultured to catalyze and synthesize LNTII:
The first stage is the cell growth phase:
Cells are cultured with glucose as a carbon source for accumulation of bacterial biomass and enzyme amount until reaching the logarithmic or stable phase of cell growth. Then, the strains are cultured in a solid medium after scratching. After 2-3 days of culture at 30°C, single colonies are selected and inoculate into 1.5mL liquid medium for further culture at 30°C and 200rpm until OD₆₀₀ =1. The cultured colonies are then inoculated at a seed volume of 2% into 5L liquid culture medium (10L fermentation tank) for incubation overnight at 30°C and 200rpm. Subsequently, the cultured colonies are inoculated at a seed volume of 2% into a 50mL liquid culture shake flask, and shaken at 30°C and 200rpm for accumulation of bacterial biomass.

The second stage is the product synthesis phase: After shaking culture at 30°C and 200rpm for 40 h, fed-batch fermentation is started and lasted for 72 h. After 72 h of fermentation, the fermentation broth is centrifuged and a high-pressure homogenizer is used to for cell disruption. After protein removal through centrifugation, the supernatant is collected and mixed with the fermentation broth to obtain a LNTII-containing solution.

The specific situation of the culture medium is as follows:
Solid culture medium: 20g/L tryptone, 10g/L yeast extract, 20g/L glucose, 20g/L agar powder, sterilized by high-temperature steam at 115°C for 30 min before use.

Liquid culture medium: 20g/L tryptone, 10g/L yeast extract, 20g/L glucose, 8g/L lactose, as well as potassium dihydrogen phosphate, magnesium sulfate, ammonium sulfate and manganese sulfate at their respective final concentrations of 5mM, sterilized by high-temperature steam at 115°C for 30 min before use.

Supplements: 2g/L lactose, 20g/L glucose, as well as potassium dihydrogen phosphate, magnesium sulfate, ammonium sulfate and manganese sulfate at their respective final concentrations of 5mM.

LNTII concentrate solution and LNTII amorphous powder were prepared:
The fermentation broth containing LNTII obtained above is taken for decolorization with anion-cation exchange resin, desalination, and purification with chromatography resin to obtain a purified solution. Then, the obtained purified solution is concentrated to a concentration of 466g/L of LNTII in the solution to obtain a concentrated LNTII-containing solution. One part of the concentrated solution is for further use and the other part for spray-drying to obtain LNTII solid powder. The obtained powder is amorphous by X-ray diffraction (XRD). The content of LNTII in the amorphous powder is determined to be 91.24% by HPLC, and the melting point is 132.3°C.

The obtained concentrated LNTII-containing solution and the LNTII amorphous powder obtained by spray-drying are used as raw materials in the following examples.

Example 1. An amount of 5g of LNTII amorphous powder is taken and added with 10g (10mL) of purified water to dissolve at 50°C (completely dissolved). After dissolution and becoming clear, the solution is cooled to 25°C at a cooling rate of 0.5°C/min. The cooled solution is then added with 0.06g of crystal seeds and 50g of anhydrous ethanol (about 63.3mL) with a fed-batch rate of 0.15mL/min. The obtained solution is stirred for 12 h of crystallization. The processed solution is then filtered, and the obtained filter residue is washed with anhydrous ethanol pre-cooled at 0°C, and dried at 55°C to obtain LNTII crystalline form, which are called crystalline form B of LNTII (hereinafter referred to as crystalline form B). The purity determined by HPLC is 96.8%. The recovery rate of LNTII is 90.1%.

X-ray diffraction (XRD) is carried out on the raw material LNTII amorphous powder (obtained by spray-drying) before crystallization and the crystalline form obtained in this Example according to the XRD method (the second method of powder XRD) specified in Appendix 0451 of the Chinese Pharmacopoeia (2020 Edition). The X-ray diffraction pattern of the raw material LNTII amorphous powder before crystallization is shown in the FIG. 1. The X-ray diffraction pattern of the crystalline form obtained in this Example is shown in the FIG. 2, with characteristic peaks of the crystal. The peak positions and intensities are shown in Table 1.

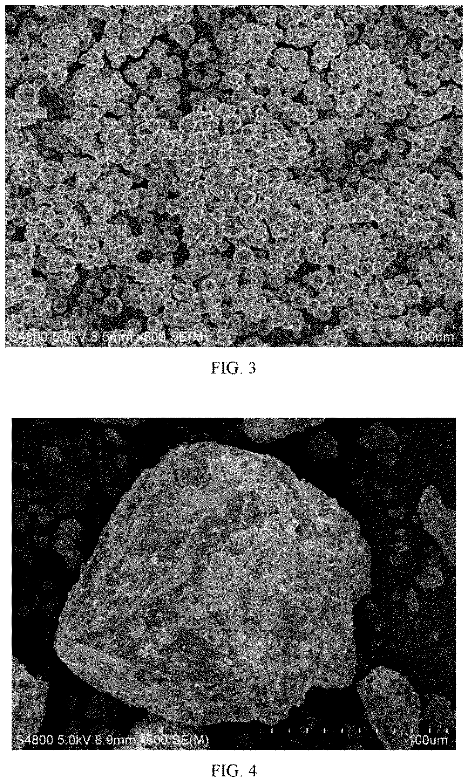

The morphology of the raw material LNTII amorphous powder before crystallization and the crystalline form obtained by the present invention are tested according to JY/T0584-2020 "General Rules for Analytical Methods of Scanning Electron Microscopy". The electron microscopy scanning pattern of the raw material LNTII amorphous powder before crystallization is shown in the FIG. 3; and the electron microscope scanning pattern of crystalline form B of LNTII obtained in this Example is shown in the FIG. 4.

The melting point of the crystalline form obtained in this Example is detected to be 190 °C, according to the method specified in GB/T21781-2008 "Test Method for Melting Point and Melting Range of Chemical Products - Capillary Tube Method".

**Table 1**

| 2θ | Relative peak intensity |
|---|---|
| 3.9776 | 242.57 |
| 6.9790 | 203.67 |
| 8.3047 | 380.94 |
| 9.6046 | 627.53 |
| 11.6235 | 480.26 |
| 12.715 | 202.62 |
| 18.6752 | 738.79 |
| 19.2522 | 771.91 |
| 19.8902 | 771.86 |
| 20.7252 | 701.34 |
| 21.0055 | 447.16 |
| 21.5672 | 387.69 |
| 26.9840 | 156.27 |
| 29.0615 | 228.05 |
| 29.5461 | 96.05 |
| 31.0814 | 177.32 |
| 31.8467 | 51.08 |
| 33.3570 | 93.7 |

Example 2. An amount of 5g of LNTII amorphous powder is taken and added with 25g (25mL) of purified water to dissolve at 40°C (completely dissolved). After dissolution and becoming clear, the solution is cooled to 15°C. The cooled solution is then added with 0.02g of crystal seeds and 50g of anhydrous ethanol (about 63.3mL) with a fed-batch rate of 0.07mL/min. The obtained solution is stirred for 20 h of crystallization. The processed solution is then filtered, and the obtained filter residue is washed with anhydrous ethanol pre-cooled at 8°C, and dried at 60°C to obtain LNTII crystalline form. Its X-ray diffraction pattern is the same as the crystalline form obtained in Example 1, which is crystalline form B of LNTII. The melting point is 192°C, and the purity determined by HPLC is 96.5%. The recovery rate of LNTII is 90.3%.

Example 3. An amount of 5g of LNTII amorphous powder is taken and added with 12g (12mL) of purified water to dissolve at 40°C (completely dissolved). After dissolution and becoming clear, the solution is cooled to 15°C. The cooled solution is then added with 50g of 95% (volume ratio) ethanol (approximately 61.7mL) with a fed-batch rate of 0.12mL/min. The obtained solution is stirred for 48 h of crystallization. The processed solution is then filtered, and the obtained filter residue is washed with 95% ethanol pre-cooled at 10°C, and dried at 60°C to obtain LNTII crystalline form. Its X-ray diffraction pattern is the same as the crystalline form obtained in Example 1, which is crystalline form B of LNTII. The melting point is 190°C, and the purity determined by HPLC is 96.9%. The recovery rate of LNTII is 88.3%.

Example 4. An amount of 5g of LNTII amorphous powder is taken and added with 20g (20mL) of purified water to dissolve at 55°C (completely dissolved). After dissolution and becoming clear, the solution is cooled to 10°C. The cooled solution is then added with 70g of anhydrous ethanol (approximately 88.6mL) with a fed-batch rate of 0.18mL/min. The obtained solution is stirred for 48 h of crystallization. The processed solution is then filtered, and the obtained filter residue is washed with anhydrous ethanol pre-cooled at 0-8°C, and dried at 65°C to obtain LNTII crystalline form. Its X-ray diffraction pattern is the same as the crystalline form obtained in Example 1, which is crystalline form B of LNTII. The melting point is 192°C, and the purity determined by HPLC is 96.1 %. The recovery rate of LNTII is 93.2%.

Example 5. The purified solution (LNTII at concentration of 466g/L) is taken and cooled to 25°C. An amount of 10mL of the cooled purified solution is taken and added with 40g of anhydrous ethanol (approximately 50.6mL) with a fed-batch rate of 0.25mL/min. The obtained solution is placed under static condition for 48 h of crystallization. The processed solution is then filtered, and the obtained filter residue is washed with anhydrous ethanol pre-cooled at 5°C, and dried at 55°C to obtain LNTII crystalline form. Its X-ray diffraction pattern is the same as the crystalline form obtained in Example 1, which is crystalline form B of LNTII. The melting point is 189°C, and the purity determined by HPLC is 93.4%. The recovery rate of LNTII is 95.0%.

Example 6. The LNTII-containing concentrated solution (LNTII at concentration of 466g/L) is taken and cooled to 15°C. An amount of 10mL of the cooled concentrated solution is taken and added with 0.06g of crystal seeds, and 30g of 95% ethanol (approximately 37mL) with a fed-batch rate of 0.18mL/min. The obtained solution is placed under static condition for 16 h of crystallization. The processed solution is then filtered, and the obtained filter residue is washed with anhydrous ethanol pre-cooled at 0°C, and dried at 60°C to obtain LNTII crystalline form. Its X-ray diffraction pattern is the same as the crystalline form obtained in Example 1, which is crystalline form B of LNTII. The melting point is 185°C, and the purity determined by HPLC is 92.4%. The recovery rate of LNTII is 91.8%.

Example 7. The LNTII-containing concentrated solution (LNTII at concentration of 466g/L) is taken and cooled to 20°C. An amount of 10mL of the cooled concentrated solution is taken and added with 0.06g of crystal seeds, and 45g of anhydrous ethanol (approximately 55.6mL) with a fed-batch rate of 0.20mL/min. The obtained solution is placed under static condition for 12 h of crystallization. The processed solution is then filtered, and the obtained filter residue is washed with anhydrous ethanol pre-cooled at 0°C, and dried at 65°C to obtain LNTII crystalline form. Its X-ray diffraction pattern is the same as the crystalline form obtained in Example 1, which is crystalline form B of LNTII. The melting point is 187°C, and the purity determined by HPLC is 93.8%. The recovery rate of LNTII is 94.5%.

Example 8. An amount of 5g of LNTII amorphous powder is taken and added with 15g (15mL) of purified water to dissolve at 45°C (completely dissolved). After dissolution and becoming clear, the solution is cooled to 25°C. The cooled solution is then added with 0.06g of crystal seeds for 0.5-1 h of crystallization; cooled to 15°C at a cooling rate of 0. 1°C/min; at this temperature, the solution is added with 45g of anhydrous ethanol (approximately 57mL) with a fed-batch rate of 0.15mL/min. The obtained solution is stirred for 18 h of crystallization. The processed solution is then filtered, and the obtained filter residue is washed with anhydrous ethanol pre-cooled at 5°C, and dried at 55°C to obtain LNTII crystalline form. Its X-ray diffraction pattern is the same as the crystalline form obtained in Example 1, which is crystalline form B of LNTII. The melting point is 191°C, and the purity determined by HPLC is 96.5% The recovery rate of LNTII is 92.9%.

### Example 9. Moisture absorption measurement

### Step 1. Processing of the glass culture dish

On the day before the constant temperature and moisture test, two dry glass culture dishes (without covers) are taken and placed in a Constant Temperature & Humidity Chamber at 25±1°C and relative humidity of 75±2% for 4 h. The processed dishes are taken out and weighed accurately. The experiment is repeated until the weight is constant, and the data is recorded in Table 3.

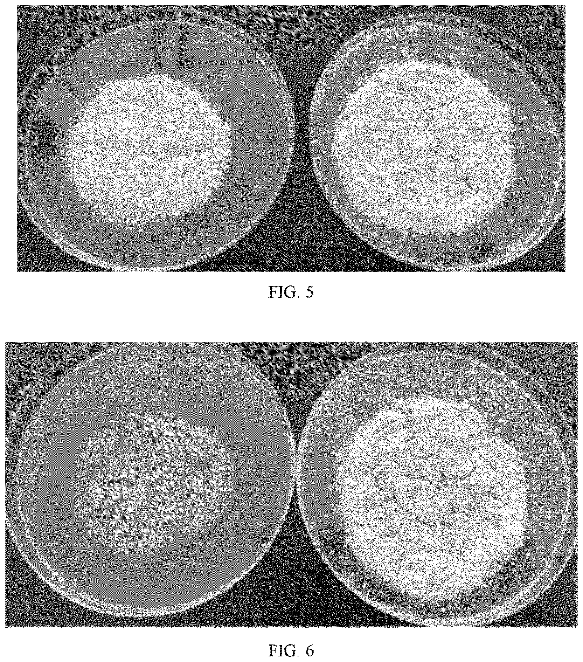

Step 2. The raw material LNTII solid amorphous powder and the crystalline form B of LNTII obtained from Example 1 are weighed separately, placed in a glass culture dish without cover with constant weight in Step 1. The culture dish with samples added are placed in a Constant Temperature & Humidity Chamber at 25°C and relative humidity of 75%. After 5 days, the samples are taken out to observe the appearance changes, weigh, and calculate the moisture absorption. The results are recorded in Table 2 and Table 3, respectively. The results of samples morphology before and after moisture absorption are shown in FIG. 5 and FIG. 6, respectively.

**Table 2**

| Samples | Appearance before experiment | Appearance on Day 5 |
|---|---|---|
| LNTII amorphous powder | Off-white powder | Faint yellow, pasty, and hardened |
| Crystalline form B of LNTII | Off-white | Off-white color, without significant changes in appearance |

**Table 3**

| Samples | Before experiment | | | End of experiment | | |
|---|---|---|---|---|---|---|
| | Weight of the culture dish (After reaching constant weight in Step 1), g | Weight of the culture dish+sample, g | Weight of the sample, g | Weight of the culture dish+sample, g | Weight gain after moisture absorption | Moisture absorption rate, % |
| LNTII amorphous powder | 6.5939 | 7.5951 | 1.0012 | 7.8902 | 0.2951 | 29.47 |
| Crystalline form B of LNTII | 6.9346 | 7.9350 | 1.0004 | 8.0967 | 0.1617 | 16.16 |

| | | | | | | |
|---|---|---|---|---|---|---|
| Note: Moisture absorption rate %=(Weight gain after moisture gain/sample weight)×100% | | | | | | |

The appearance in Table 2 and the results in Table 3 indicate that the stability of crystalline form B of LNTII described in the present invention is better than that of amorphous powder, which is beneficial for the long-term storage of LNTII products and their application in food and medicine. It can also reduce the packaging cost of LNTII and extend the shelf life of the product.

### Example 10. Moisture absorption study

According to the method described herein in Example 9, the crystalline form B of LNTII prepared according to Examples 1-8 is placed in a glass culture dish without cover with constant weight in Step 1. The culture dish with samples added are placed in a Constant Temperature & Humidity Chamber at 25°C and relative humidity of 75%. After 5 days, the samples are taken out to observe the appearance changes, weigh, and calculate the moisture absorption. The results are respectively recorded in Table 4.

**Table 4. Moisture absorption study on crystalline form B of LNTII obtained from Examples 1-8**

| | Appearance | Moisture absorption rate, % |
|---|---|---|
| Crystalline form B in Example 1 | Off-white, without significant changes | 14.02 |
| Crystalline form B in Example 2 | Off-white, without significant changes | 14.36 |
| Crystalline form B in Example 3 | Off-white, without significant changes | 13.57 |
| Crystalline form B in Example 4 | Off-white, without significant changes | 13.81 |
| Crystalline form B in Example 5 | Off-white, without significant changes | 13.79 |
| Crystalline form B in Example 6 | Off-white, without significant changes | 13.32 |
| Crystalline form B in Example 7 | Off-white, without significant changes | 13.04 |
| Crystalline form B in Example 8 | Off-white, without significant changes | 13.61 |

### Example 11. Stability study

An amount of 10g of raw material before crystallization (i.e. LNTII amorphous powder obtained by spray-drying) and 10g of crystalline form B of LNTII prepared by the method described herein in Examples 1-8 are weighed respectively, sealed with aluminum compound packing film, and placed in a Constant Temperature & Humidity Chamber under the test conditions of at constant temperature of 40°C and relative humidity of 75%. Samples are taken at the end of the 1^{st} month, the 2^{nd} month, and the 3^{rd} month to test the content of LNTII and observe its appearance. The results are recorded in Table 5.

**Table 5. Stability study on amorphous LNTII and crystalline form B of LNTII**

| | Day 0 | | At the end of the 1^{st} month | | At the end of the 2^{nd} month | | At the end of the 3^{rd} month | |
|---|---|---|---|---|---|---|---|---|
| | Conte nt, % | Appearan ce | Conte nt, % | Appearan ce | Conte nt, % | Appearan ce | Conte nt, % | Appearan ce |
| LNTII amorphous powder | 91.24 | Off-white | 91.01 | Off-white | 90.86 | Off-white | 90.01 | Faint yellow |
| Crystalline form B in Example 1 | 96.8 | Off-white | 96.81 | Off-white | 96.78 | Off-white | 96.76 | Off-white |
| Crystalline form B in Example 2 | 96.5 | Off-white | 96.44 | Off-white | 96.42 | Off-white | 96.38 | Off-white |
| Crystalline form B in | 96.9 | Off-white | 96.92 | Off-white | 96.93 | Off-white | 96.84 | Off-white |
| Example 3 | | | | | | | | |
| Crystalline form B in Example 4 | 96.1 | Off-white | 96.13 | Off-white | 96.06 | Off-white | 96.07 | Off-white |
| Crystalline form B in Example5 | 93.4 | Off-white | 93.56 | Off-white | 93.48 | Off-white | 93.34 | Off-white |
| Crystalline form B in Example 6 | 92.4 | Off-white | 92.43 | Off-white | 92.39 | Off-white | 92.31 | Off-white |
| Crystalline form B in Example 7 | 93.8 | Off-white | 93.77 | Off-white | 93.81 | Off-white | 93.75 | Off-white |
| Crystalline form B in Example 8 | 96.5 | Off-white | 96.48 | Off-white | 96.49 | Off-white | 96.46 | Off-white |

Data explanation in Table 5: During the stability experiment on crystalline form B obtained from Examples 1-8 of the present invention, the content and appearance of LNTII did not change; while the content of the raw material LNTII solid powder showed a decreasing trend, and the color gradually changed to faint yellow.

It shall be noted that the above embodiments are only used to illustrate the technical solution of the present invention and not limitation. Although the present invention has been described in detail with reference to the embodiments thereof, common technicians in the art may modify or equivalently replace the technical solution of the present invention as needed, without departing from the spirit and scope of the technical solution of the present invention.

## Claims

1. A crystalline form B of lacto-N-triose II (LNTII), wherein a powder X-ray diffraction pattern exhibits characteristic peaks at 2θ±0.2° at 2θ ± 0.2° values of 3.98, 6.98, 8.30, 9.60, 18.68, 19.25, 19.89 and 20.72.

2. The crystalline form B of LNTII according to claim 1, wherein the powder X-ray diffraction pattern exhibits characteristic peaks at 2θ±0.2° values of 3.98, 6.98, 8.30, 9.60, 11.62, 18.68, 19.25, 19.89, 20.72, 21.00 and 21.56.

3. The crystalline form B of LNTII according to claim 1, wherein the powder X-ray diffraction pattern exhibits characteristic peaks at 2θ±0.2° values of 3.98, 6.98, 8.30, 9.60, 11.62, 12.72, 18.68, 19.25, 19.89, 20.72, 21.00, 21.56 and 29.06.

4. The crystalline form B of LNTII according to claim 1, wherein the powder X-ray diffraction pattern exhibits characteristic peaks at 2θ±0.2° values of 3.98, 6.98, 8.30, 9.60, 11.62, 12.72, 18.68, 19.25, 19.89, 20.72, 21.00, 21.56, 26.98, 29.06, 29.54, 31.08, 31.84 and 33.36.

5. The crystalline form B of LNTII according to any one of claims 1 to 4, wherein having a melting point ranging from 185°C to 192°C.

6. A method for preparing the crystalline form B of LNTII according to any one of claims 1 to 5, comprising: mixing a solution containing LNTII with an organic solvent to obtain crystalline form B of LNTII; preferably, the organic solvent is ethanol, wherein the ethanol is anhydrous ethanol or an ethanol aqueous solution.

7. The method according to claim 6, wherein the mass of the organic solvent in a mixed solution obtained by mixing the solution containing LNTII with the organic solvent is greater than the mass of water; preferably, the mass ratio of water to the organic solvent is 1:2-5; more preferably is 1:3-4; preferably, the organic solvent is anhydrous ethanol or an ethanol aqueous solution.

8. The method according to claim 6, wherein the method further comprises adding seed crystals to the solution containing LNTII, allowing crystal growth for more than 0.5 hours, and then mixing with the organic solvent to obtain crystalline form B of LNTII.

9. A use of the crystalline form B of LNTII according to any one of claims 1 to 5 as an intermediate or end product.
